(19) Europäisches Patentamt

European Patent Office

Office européen des brevets

(11) **EP 1 029 839 A1**

(12) **EUROPÄISCHE PATENTANMELDUNG**

(43) Veröffentlichungstag:
**23.08.2000 Patentblatt 2000/34**

(51) Int. Cl.$^7$: **C07C 11/02**

(21) Anmeldenummer: **99126213.0**

(22) Anmeldetag: **30.12.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **17.02.1999 DE 19906518**

(71) Anmelder:
**Oxeno Olefinchemie GmbH**
**45764 Marl (DE)**

(72) Erfinder:
**Wiese, Klaus-Diether Dr.**
**45721 Haltern (DE)**

(54) **Verfahren zur Fraktionierung von Dibuten**

(57) Die Erfindung betrifft ein Verfahren zur Auftrennung von Dibutenen in eine n-Octen-haltige und eine Dimethylhexen-haltige Fraktion.

Die Fraktionen können getrennt zu den entsprechenden $C_9$-Carbonsäuren und $C_9$-Alkoholen (Isononanole) weiterverarbeitet werden.

Folgeprodukte der $C_9$-Carbonsäuren sind z. B. Vinylester.
Folgeprodukte der $C_9$-Alkohole sind Weichmacher.

EP 1 029 839 A1

**Beschreibung**

Die Erfindung betrifft ein Verfahren zur Fraktionierung eines Dibutengemisches sowie die Verwendung der Dibutenfraktionen.

Unter Dibuten versteht man Gemische isomerer $C_8$-Olefine, die durch Dimerisierung von n-Butenen oder von n-Buten-haltigen $C_4$-Strömen erhalten werden. Besonders günstig ist es, das sogenannte Raffinat II oder Raffinat III, das preiswert aus der Verarbeitung roher Crack-$C_4$-Schnitte zugänglich ist, dafür einzusetzen.

Zur Gewinnung von Raffinat II oder III wird aus rohen Crack-$C_4$-Schnitten im ersten Schritt Butadien entfernt. Dies geschieht entweder durch Extraktion des Butadiens oder dessen Selektivhydrierung zu den linearen Butenen. In beiden Fällen wird ein praktisch butadienfreier $C_4$-Schnitt erhalten, das Raffinat I. Im zweiten Schritt wird Isobuten aus dem $C_4$-Strom entfernt, z. B. durch Herstellung von Methyl-tert.-butylether (MTBE) durch Umsetzung mit Methanol. MTBE ist eine gefragte Kraftstoffkomponente. Andere Möglichkeiten sind die Umsetzung des Isobutens aus dem Raffinat I mit Wasser zu TBA (tertiär-Butanol) oder die sauer katalysierte Oligomerisierung des Isobutens zu Diisobuten. Der jetzt isobutenfreie $C_4$-Schnitt, das Raffinat II, enthält wie gewünscht die linearen Butene und gegebenenfalls Butane. Optional kann auch noch das 1-Buten destillativ abgetrennt werden, der 1-Butenfreie Schnitt wird dann als Raffinat III bezeichnet.

Für die Herstellung von Di-n-Buten kann alternativ Raffinat II oder Raffinat III eingesetzt werden. Die Verwendung anderer technischer $C_4$-Ströme, wie z. B. solche aus Fischer-Tropsch-Olefinen, ist möglich. Entscheidend ist, daß im wesentlichen nur lineare Butene darin enthalten sind

Die Oligomerisierung solcher n-Buten-haltiger $C_4$-Ströme zu im wesentlichen $C_8$-Olefine enthaltenden Gemischen ist grundsätzlich bekannt. Im Prinzip gibt es drei Verfahrensvarianten. Lange bekannt ist die Oligomerisierung an sauren Katalysatoren (Verfahren A), wobei technisch z. B. Zeolithe oder Phosphorsäure auf Träger eingesetzt werden. Hierbei werden Isomerengemische von verzweigten Olefinen erhalten, die im wesentlichen Dimethylhexene darstellen (WO 92/13818). Ein ebenfalls weltweit ausgeübtes Verfahren ist die Oligomerisierung mit löslichen Ni-Komplexen, bekannt als DIMERSOL-Verfahren (Verfahren B) (B. Cornils, W.A. Herrmann, Applied Homogenous Catalysis with Organometallic Compounds, Seite 261-263,Verlag Chemie 1996). Schließlich ist noch die Oligomerisierung an Nickel-Festbett-Katalysatoren zu erwähnen, wie z. B. das Verfahren der OXENO-GmbH. Das Verfahren hat Eingang in die Literatur als OCTOL-Prozeß (Verfahren C) (Hydrocarbon Process., Int. Ed. (1986) 65 (2. Sect.1), Seite 31-33) gefunden.

Die so erhaltenen Dibutene sind begehrte Einsatzstoffe in der chemischen Industrie. So können beispielsweise durch Hydroformylierung um ein Kohlenstoffatom längere Aldehyde gewonnen werden - im Fall von Dibuten somit $C_9$-Aldehyde - die ihrerseits wieder für wichtige technische Produkte Einsatz finden. Beispiele sind die Hydrierung der Aldehyde zu den Alkoholen und ihre Umsetzung mit Carbonsäuren zu Estern. So führt die Veresterung der Alkohole mit Phthalsäureanhydrid zu Diisononylphthalaten, die sehr begehrte Weichmacher in der kunststoffverarbeitenden Industrie sind. Technisch wichtig und ausgeübt ist des weiteren die Oxidation der Aldehyde zu den entsprechenden Carbonsäuren, die unter anderem zu öllöslichen Metallsalzen umgesetzt werden. Diese werden beispielsweise als Trocknungsbeschleuniger für Lacke (Sikkative) oder Stabilisatoren für PVC eingesetzt.

Eine weitere beispielhafte technische Anwendung ist die Umsetzung von Olefinen (Dibutenen) unter Katalyse von starken Säuren mit Kohlenmonoxid und Wasser zu den um ein Kohlenstoffatom längeren Carbonsäuren, die unter dem Namen KOCH-Reaktion Eingang in die Literatur gefunden hat. Hier werden tertiäre verzweigte Carbonsäuregemische erhalten, die wegen ihrer verzweigten Natur wiederum sehr gut geeignet sind zur Herstellung der zuvor erwähnten Metallsalze. Ein besonders wichtiger Einsatz der tertiären Carbonsäuren ist die Umsetzung mit Acetylen zu Vinylestern, die als Comonomere zur inneren Weichmachung von Polymeren dienen. Copolymere von Vinylestern tertiärer Carbonsäuren mit Vinylacetat sind beispielsweise die Basis für wasserdispergierbare umweltfreundliche Farben und Lacke und energiesparende Wärmeschutzputze von Gebäuden.

Dibuten ist kein einheitlicher Stoff, sondern ein Gemisch von vielen Strukturisomeren, die sich ihrerseits wieder aus praktisch allen Doppelbindungsisomeren in unter-schiedlichen Anteilen zusammensetzen, wobei viele der Doppelbindungsisomeren noch zusätzlich eine cis/trans-Isomerie zeigen. Diese Konstitutions- und Konfigurationsisomere können je nach Herstellverfahren in unterschiedlichen Anteilen vorliegen.

Wird Dibuten ausgehend von Raffinat II oder III hergestellt, erhält man Olefingemische von im wesentlichen unverzweigten, einfach verzweigten und zweifach verzweigten Grundstrukturen. Die folgenden Daten dienen nur der Orientierung, da je nach Verfahrensbedingungen wechselnde Anteile der einzelnen Strukturgruppen erhalten werden.

Ein Maß für den Verzweigungsgrad ist der Isoindex. Er ist definiert durch die Anzahl der Verzweigungen pro Molekül. Demnach haben lineare Octene (n-Octene) einen Isoindex von 0, Methylheptene einen von 1 und Dimethylhexene einen von 2. Bei der Berechnung des Isoindex von Gemischen sind die Massenanteile der einzelnen Verbindungsgruppen zu berücksichtigen.

|  | A Zeolithkatalyse | B Dimersol | C Octol |
|---|---|---|---|
| n-Octene | ~ 0% | ~ 6 % | ~ 13 % |
| 3-Methyl-Heptene | ~ 5 % | ~ 59 % | ~ 62 % |
| 3,4-Dimethylhexene | ~ 70 % | ~ 34 % | ~ 24 % |
| Sonstige $C_8$-Olefine | ~ 25 % | ~ 1 % | ~ 1 % |
| Isoindex | >1,9 | ≅1,29 | ≅1,12 |

**Tabelle 1:** Typische Strukturverteilung in Dibutenen, jeweils aus unterschiedlichen Herstellverfahren, ausgehend von Raffinat III

Nimmt man an Stelle von Raffinat II oder Raffinat III andere noch Isobuten enthaltende $C_4$-Schnitte wie das Raffinat I, werden darüber hinaus eine Fülle weiterer, noch verzweigterer Strukturen gebildet, im wesentlichen Trimethylhexene wie 2,2,4-Trimethyl-Pentene; 2,2,3-Trimethyl-Pentene; 2,3,4 Trimethyl-Pentene; 2,3,3-Trimethyl-Pentene und andere mehr. Solche Dibutene mit einem Isoindex von größer 2 sind auch unter dem Namen "Codibutylen" bekannt.

Die anwendungstechnischen Eigenschaften der aus Dibuten hergestellten Folgeprodukte sind oftmals von der Zusammensetzung und insbesondere vom Verzweigungsgrad des eingesetzten Olefins abhängig. Dies kann ganz extreme Formen annehmen, wie an folgenden Beispielen zu erkennen ist.

Ein wichtiges Anwendungsgebiet von Dibutenen ist die Herstellung von $C_9$-Alkoholen, die ihrerseits mit Carbonsäuren verestert werden. So erhält man aus Dibuten Isononanolgemische und durch deren Veresterung mit Phthalsäureanhydrid Isononylphthalate; diese werden als Weichmacher in Kunststoffe eingesetzt.

Der Verzweigungsgrad der Isononylketten der Phthalate steht in einem engen Zusammenhang mit dem Verzweigungsgrad des eingesetzten Olefins, so daß die Eigenschaften der Phthalate wesentlich durch die Struktur des eingesetzten Olefingemisches mitbestimmt sind.

| Rohstoff | Oligomerisierungsverfahren | Hydroformylierungsverfahren | Viskositäten der Isononylphthalate (20 °C) |
|---|---|---|---|
| Raffinat I | A | Co-HD | ≅165 mPa s |
| Raffinat II oder III | A | Co-HD | 116-120 mPa s |
| Raffinat II oder III | B oder C | Co-HD | 70-85 mPa s |
| Raffinat II oder III | B oder C | Rh-HD | 90-100 mPa s |

**Tabelle 2:** Vergleich typischer dynamischer Viskositäten technisch eingesetzter

Nonylphthalate, mit

Co-HD: Klassisches Kobalt-Hochdruckverfahren, 200-300 bar, 140-180 °C

Rh-HD:Rhodium-Hochdruckverfahren, 150-300 bar, 120-130 °C, unmodifizierter

oder mit Triphenylphosphinoxid modifizierter Rhodiumkatalysator

Andere anwendungstechnische Eigenschaften der Weichmacher sind ebenfalls stark von deren Verzweigungsgrad abhängig. Wadey et al. zeigen in J. Vinyl Techn. (1990) 208-211 die deutliche Abhängigkeit der Weichmachereigenschaften von Dinonylphthalaten vom Verzweigungsgrad des Nonylrestes.

Die in der Polymerisation von Vinylacetaten häufig als Comonomer eingesetzten Vinylester von tertiären Carbonsäuren sind ebenfalls ein Beispiel für eine Abhängigkeit der Weichmachereigenschaften einer Verbindung von dessen Verzweigungsgrad. Ein Maß für die weichmachende Wirkung des Comonomeren ist der Glaspunkt Tg [°C] des Homopolymeren. Bei völlig gleicher Summenformel kann der Glaspunkt über eine große Spanne in Abhängigkeit von der Struktur des eingesetzten Olefins variieren. In der Literatur findet man beispielsweise für Vinylester von $C_9$-Carbonsäuren:

| Basisolefin | Vinylester (VE) von | Tg [°C] |
|---|---|---|
| 2,3,4-Trimethyl-Penten | 2,3-Dimethyl-2-Isopropyl-Butansäure | 119 |
| 2,2,3-Trimethyl-Penten | 2-Ethyl-2,3,3-Trimethyl-Butansäure | 115 |
| Diisobuten | kommerziell erhältlicher Vinylester | 70 (60)* |
| 2,2,4-Trimethyl-Penten | 2,2,4,4-Tetramethyl-Pentansäure | 55 |
| 2,4-Dimethyl-Hexen | 2,2,4-Trimethyl-Hexansäure | 10 |

**Tabelle 3:** (H. P. H. Scholten, J. Vermeulen, W.J. van Westrenen, Recent Developments in Latices based on Vinyl Esters of branched Monocarboxylic Acids, Seventh International Conference Water-Borne Coatings,London 1987;*W.Lau, VeoVa, a Vinyl Ester Monomer, Polymers Dotcom Magazine, 2(2), Februar 1996)

Bei Weichmachern kommt es somit in der Regel auf eine möglichst geringe Verzweigung der Ausgangsolefine an, wie am Beispiel der Phthalate und Vinylester gezeigt worden ist.

Dies bedeutet jedoch nicht, daß höher verzweigte Olefine wertlos wären, entscheidend ist die richtige Wahl des Einsatzgebietes. So sind beispielsweise Metallsalze hoch verzweigter Carbonsäuren, erhältlich aus entsprechend hoch verzweigten Olefinen, wegen der Abschirmung der polaren Carboxylgruppe besonders gut öllöslich und damit besonders gut für den Einsatz zur Herstellung von Sikkativen und PVC-Stabilisatoren geeignet. Ein generelles Einsatzgebiet für hoch verzweigte Olefine sind sauer katalysierte Reaktionen, wie z. B. die säurekatalysierte Alkylierung von Phenolen. Dabei können wesentlich bessere Ausbeuten als mit geringer verzweigten oder linearen Olefinen erzielt werden, da sie besonders leicht tertiäre Carbeniumionen bilden können.

Die große wirtschaftliche Bedeutung der Dibutene und ihrer Folgeprodukte sowie die starke Strukturabhängigkeit ihrer anwendungstechnischen Eigenschaften legte daher eine Auftrennung des Dibutengemisches nahe.

Eine destillative Auftrennung des Dibutengemisches ist aber auf Grund der geringen Siedepunktsunterschiede nur in analytischem Maßstab - und selbst hier nicht vollständig - möglich. Dies ist somit nicht wirtschaftlich. Zur Verdeutlichung sei darauf hingewiesen, daß im Siedebereich des Dibutens von 104 bis 125 °C bei Normaldruck bisher etwa 40 Komponenten nachgewiesen worden sind.

Großtechnisch werden daher zur Auftrennung von $C_8$-Olefinen verschiedene andere Verfahren eingesetzt wie Adsorptionsverfahren, Destillation mit Schleppmitteln oder Trennverfahren mit vorgeschalteten Isomerisierungsschritten.

In US-PS 5 262 015 wird die Abtrennung von 1-Octen von weiteren $C_8$-Olefinisomeren durch azeotrope Destillation mittels eines Schleppmittels beschrieben.

Als Schleppmittel kommen z. B. Ethylacetat und Amylmethylether zum Einsatz. Dieses Verfahren ermöglicht jedoch nur die Abtrennung von in großem Überschuß vorliegenden 1-Octen und erfordert den Einsatz von einem Teil Schleppmittel auf einen Teil Isomerengemisch. In Dibuten ist hingegen der Anteil von 1-Octen sehr gering, es liegen vorwiegend innenständige Doppelbindungen vor. Eine großtechnische Anwendung auf die Trennung von Dibuten scheidet somit aus.

US-PS 5 292 990 offenbart die Auftrennung von $C_8$-Olefingemischen durch das unterschiedliche Adsorptions-/Desorptionsverhalten der Isomeren an bestimmten Zeolithen. Es sind hierfür ganz spezielle, nur schwer oder gar nicht kommerziell erhältliche Zeolithe erforderlich, abgesehen davon, daß der Einsatz von derartigen Verfahren in der Regel auf analytische Trennverfahren abzielt. Eine großtechnische Anwendung scheidet somit aus oder ist zumindest sehr aufwendig und unwirtschaftlich.

Eine weitere Möglichkeit, zu Dibutengemischen mit geringerem Anteil an Isomeren zu kommen, besteht theoretisch in einer Isomerisierung der Dibutene vor dem eigentlichen Trennprozeß. Ein Beispiel für ein solches Verfahren liefert EP 0684721. Ziel der Isomerisierung ist es, bestimmte Isomere in andere, unter den gewählten Bedingungen thermodynamisch stabilere umzuwandeln und somit eine spätere Auftrennung zu vereinfachen. In der Regel sind aber höher substituierte und/oder innere Olefine thermodynamisch stabiler, so daß die für viele Zwecke wichtigen weniger substituierten und/oder endständigen Olefine verloren gehen. Eine direkte Auftrennung des Isomerengemisches unter Erhalt der erwünschten Strukturen ist somit mit kombinierten Isomerisierungs-/Trennverfahren großtechnisch nicht möglich.

Zusammenfassend ist festzustellen, daß in der Literatur keine großtechnisch einsetzbare und wirtschaftliche Methode existiert, um Dibutengemische aufzutrennen.

Die Eigenschaften einzelner Dibutenisomere, so auch die Siedepunkte, sind häufig sehr ähnlich. Eine weitgehende Auftrennung in Einzelisomere ist somit kaum möglich, aber wirtschaftlich auch nicht unbedingt erforderlich. Im Gegenteil wäre es wünschenswert, mit möglichst wenig apparativem und energetischem Aufwand Olefingemische zu gewinnen, die für die Herstellung von Produkten mit bestimmten Eigenschaften geeignet sind.

Der Erfindung lag daher die Aufgabe zugrunde, ein Verfahren zur Auftrennung von Dibutengemischen zu entwickeln.

Überraschenderweise wurde gefunden, daß die Fraktionierung von Dibutenen, durch Auftrennung in eine praktisch n-Octen-freie (Isoindex größer als der der ursprünglichen Dibutene) und somit verzweigte Olefine enthaltende Fraktion und in eine zweite Fraktion (Isoindex kleiner als der der ursprünglichen Dibutene), die neben nahezu sämtlichen unverzweigten und einfach verzweigten Olefinen nur geringe Mengen höher verzweigter Olefine enthält, in einem einzigen Schritt gelingt.

Gegenstand der vorliegenden Erfindung ist daher ein Verfahren zur Auftrennung von Dibutenen in eine n-Octen-haltige Fraktion mit einem Isoindex von weniger als 90 % und eine Dimethylhexen-haltige Fraktion mit mehr als 110 % des Isoindex der ursprünglichen Dibutene.

Mit Hilfe des erfindungsgemäßen Verfahrens ist es nun möglich, Dibutenfraktionen herzustellen und die einzelnen Fraktionen einer gezielten Weiterverarbeitung zuzuführen. Die Folgeprodukte der Einzelfraktionen weisen, verglichen mit den Folgeprodukten des Dibutengemisches, definierte und nachhaltig verbesserte Eigenschaften auf.

Tabelle 4 zeigt beispielhaft die Isomerenverteilung einer typischen Dibutenpobe, die durch Oligomerisierung von Raffinat III nach dem Octol-Prozess hergestellt worden ist, zusammen mit den zugehörigen Massenanteilen. Als Maß für die Siedepunkte, die teilweise nicht bekannt sind, sind die gaschromatographisch ermittelten Retentionsindices, gemessen auf Squalen, angegeben. Die angegebenen Retentionsindices repräsentieren einen Siedebereich von ca. 105 bis 126 °C. Die geringen Unterschiede der Retentionsindices weisen auf sehr kleine Siedepunktsdifferenzen der Isomere hin. Dazu wechseln sich mehr und weniger verzweigte Komponenten in der Reihenfolge unsystematisch ab.

| Nr. | Retentionsindex | | Isomeres | Massenanteil |
|---|---|---|---|---|
| | auf Squalan | Sp | | der Isomeren |
| 1 | 725,4 | 105,1 °C | 4,4-Dimethyl-Hexen-1 | 0,17 % |
| 2 | * | | cis-2,4-Dimethyl-Hexen-3 | ** |
| 3 | 728,0 | | trans-2,4-Dimethyl-Hexen-3 | 0,07 % |
| 4 | 730,7 | | 2,4-Dimethyl-Hexen-2 | 0,14 % |
| 5 | 733,7 | | 2-Methyl-3-Ethyl-Penten-1 | 0,05 % |
| 6 | 734,7 | | trans —4,5-Dimethyl-Hexen-2 | 0,08 % |
| 7 | 736,0 | | cis-4,5-Dimethyl-Hexen-2 | 0,08 % |
| 8 | 738,3 | | cis-5-Methyl-Hepten-3 | 1,13 % |
| 9 | 740,1 | | 3-Methyl-Hepten-1 | 0,71 % |
| 10 | 741,0 | | trans-5-Methyl-Hepten-3 | 6,37 % |
| 11 | 749,6 | | 3-Methyl-2-Ethyl-Penten-1 | 0,93 % |
| 12 | 752,0 | | trans-3,5-Dimethyl-Hexen-2 | 0,37 % |
| 13 | 752,3 | | cis-3,5-Dimethyl-Hexen-2 | 0,10 % |
| 14 | 755,7 | | cis-3,4-Dimethyl-Hexen-2 | 4,69 % |
| 15 | 760,0 | | trans-3,4-Dimethyl-Hexen-2 | 14,41 % |
| 16 | 767,6 | | trans-5-Methyl-Hepten-2 | 9,62 % |
| 17 | 775,9 | | cis-5-Methyl-Hepten-2 | 3,56 % |
| 18 | 777,1 | | trans-3,4-Dimethyl-Hexen-3 | 2,99 % |
| 19 | 777,6 | | cis-3-Methyl-Hepten-3 | 5,21 % |
| 20 | 779,1 | 120,0 °C | 2-Ethyl-Hexen-1 | 1,07 % |
| 21 | 781,9 | 121,3 °C | n-Octen-1 | 0,10 % |

| 22 | 782,9 | | cis-3,4-Dimethyl-Hexen-3 | 2,79 % |
|---|---|---|---|---|
| 23 | 784,1 | | trans-3-Methyl-Hepten-3 | 9,77 % |
| 24 | * | 122,3 °C | trans-n-Octen-4 | 3,06 % |
| 25 | 786,6 | 122,5 °C | cis-n-Octen-4 | 0,67 % |
| 26 | 790,8 | | 2,3-Dimethyl-Hexen-2 | 0,10 % |
| 27 | 788,3 | | cis-3-Methyl-Hepten-2 | 6,58 % |
| 28 | * | 122,9 °C | cis-n-Octen-3 | 1,11 % |
| 29 | 789,1 | 123,3 °C | trans-n-Octen-3 | 5,08 % |
| 30 | 798,8 | | trans-3-Methyl-Hepten-2 | 11,30 % |
| 31 | * | 125,0°C | trans-n-Octen-2 | 5,10 % |
| 32 | 802,0 | 125,6°C | cis-n-Octen-2 | 1,88 % |
| Rest | | | Alkane und Unbekannte | 0,71 % |

**Tabelle 4:** Siedepunktsangaben aus Handbook of Chemistry and Physics, 67. Ed. 1986-1987, CRC-Press; *Die Retentionsindices liegen zwischen den benachbarten Werten. Sie konnten wegen Peaküberlagerungen nicht genau bestimmt werden; **Spuren

Dennoch gelingt eine Trennung mittels des erfindungsgemäßen Verfahrens, bevorzugt durch einen destillativen Schnitt zwischen den Verbindungen 15 und 16 der Tabelle 4.

Die geringen Siedepunktsunterschiede der Isomeren erfordern zur destillativen Trennung eine Kolonne mit einer entsprechend hohen Trennleistung bzw. der nötigen Anzahl theoretischer Böden. Dies kann durch Einbau von Kolonnenböden, Lochblechen oder Fullkörpern wie Raschigringen, Maschendrahtgeweben oder Packungen erreicht werden Besonders bevorzugt sind wegen der hohen Trennleistung bei geringem Differenzdruck Gewebepackungen, wie z. B. CY-Packungen der Fa. Sulzer.

Eine vollständige isomerenreine Auftrennung des Dibutens ist für viele Folgeprodukte des Dibutens nicht erforderlich; in der Praxis hat sich eine Fraktionierung in eine hauptsächlich gering verzweigte Olefine (Isoindex kleiner 90 % der der ursprünglichen Dibutene) enthaltende und in eine hauptsächlich höher verzweigte Olefine (Isoindex größer 110 % der der ursprünglichen Dibutene) enthaltende Fraktion als völlig ausreichend erwiesen.

Bevorzugt liegt der Isoindex der n-Octen-haltigen Fraktion unter 1,0, besonders bevorzugt unter 0,9. Dies bedeutet in beiden Fällen, daß der Isoindex der Dimethylhexen-haltigen Fraktion größer als der Isoindex der ursprünglichen Dibutene (Einsatzolefin) wird.

Die Fraktionierung der Dibutene im erfindungsgemäßen Verfahren kann durch eine kontinuierliche Destillation durchgeführt werden. Die Destillation kann in einem weiten Druckbereich, d. h. sowohl unter Vakuum- als auch unter Überdruckbedingungen ausgeführt werden. Bevorzugt ist jedoch die Destillation bei Normaldruck, um den technischen Aufwand gering zu halten.

Im erfindungsgemäßen Verfahren wird die n-Octen-haltige Fraktion als Sumpfprodukt erhalten. Diese Fraktion weist bevorzugt einen Siedebereich von 110 bis 126 °C, besonders bevorzugt von 115 bis 123 °C unter Normaldruck auf.

Die Dimethylhexen-haltige Fraktion wird dagegen als Kopfprodukt erhalten. Das Kopfprodukt weist einen Siedebereich unter Normaldruck von 95 bis 120 °C, besonders bevorzugt von 105 bis 115 °C auf.

Die angegebenen Siedebereiche sind druckabhängig. Wird das erfindungsgemäße Verfahren bei anderen Drücken angewendet, so sind die bevorzugten Siedebereiche entsprechend umzurechnen.

Die mit dem erfindungsgemäßen Verfahren hergestellten Dibutenfraktionen können in Folgereaktionen eingesetzt werden, bei denen auch das unbehandelte Dibutengemisch üblicherweise verwendet wird. Hierzu zählen säurekatalysierte Reaktionen wie die KOCH-Synthese zu tertiären Carbonsäuren sowie deren Weiterverwendung zur Herstellung von Vinylestern, oder die Alkylierung von Benzolen und Phenolen, weiter die metallkatalysierte Herstellung von Aldehyden durch Hydroformylierung sowie die Weiterverarbeitung der entstandenen Aldehyde zu den entsprechenden Alkoholen und den davon abgeleiteten Weichmachern, aber auch die Oxidation der durch Hydroformylierung erhaltenen Aldehyde zu nicht tertiären Carbonsäuren.

Die wenig verzweigte n-Octen-haltige Sumpffraktion wird beispielsweise dann besonders vorteilhaft zum Einsatz kommen, wenn biologisch leicht abbaubare Produkte hergestellt werden sollen. Bekanntlich sind wenig verzweigte Alkylketten leichter biologisch abbaubar als höher verzweigte.

Des weiteren ist die geringe Verzweigung des Sumpfproduktes von besonderem Vorteil, wenn die Folgeprodukte Weichmacher sind. So erhält man durch Hydroformylierung der wenig verzweigten Sumpffraktion ein weniger verzweigtes Isononanol als bei Einsatz des nicht aufgetrennten Gemisches. Dieses ergibt nach Veresterung beispielsweise mit Phthalsäureanhydrid einen Weichmacher mit deutlich verbesserten Eigenschaften (siehe Vergleichsbeispiele).

Die erfindungsgemäß gewonnenen Dibutenfraktionen (n-Octen- und/oder Dimethylhexen-haltige Fraktion) können zur Herstellung von Isononanolen bzw. deren Ester verwendet werden. Die Isononanole werden z. B. durch Hydroformylierung der Dibutene zu den entsprechenden Aldehyden und deren Hydrierung hergestellt. Die Hydroformylierung kann wahlweise mit Kobalt-oder Rhodiumkatalysatoren durchgeführt werden. Als Beispiel seien kobaltkatalysierte Reaktionssysteme, bei denen Kobaltsalze in wäßriger Lösung mit Synthesegas ($H_2$: CO im Volumenverhältnis 1:1) zu Kobalthydridocarbonylen umgesetzt werden, genannt. Diese Kobaltkomplexe katalysieren die Reaktion der Olefine mit Synthesegas zu den entsprechenden Oxoaldehyden. Die Oxoaldehyde werden anschließend zu den gewünschten Alkoholen hydriert. Diese Prozesse sind bekannt und z. B. in DE-OS 21 39 630, DE-OS 21 06 252, DE-OS 22 44 373 oder WO 93/24438 beschrieben.

In der Regel wird die Hydroformylierung mit einer Reaktionstemperatur von 50 bis 200 °C und einem Synthesegasdruck von 100 bis 400 bar durchgeführt. Das Olefin dient - gegebenenfalls mit einem Alkohol als Lösungsvermittler - gleichzeitig als Edukt und Lösungsmittel. Nach erfolgter Hydroformylierung wird der Katalysator oxidativ zerstört, die wäßrige Phase vom Produktgemisch getrennt, die nicht umgesetzten Olefine in die Hydroformylierungsstufe zurückgefahren und die erhaltenen Oxealdehyde zu den entsprechenden Isononanolen hydriert. Die Weiterverarbeitung zu den Diisononylphthalaten erfolgt durch Veresterung mit Phthalsäureanhydrid, zum Beispiel unter der Katalyse von Butyltitanaten.

Ein weiteres Beispiel für die Verwendung der durch das erfindungsgemäße Verfahren hergestellten Dibutenfraktionen (n-Octen- und/oder Dimethylhexen-haltige Fraktion) ist die Herstellung von Nonansäuren bzw. deren Vinylestern. Hierzu wird zunächst die jeweilige Olefinfraktion mit Kohlenmonoxid in Anwesenheit starker Säuren wie Schwefelsäure oder Borfluorid-Hydraten zu tertiären Carbonsäuren umgesetzt. Die tertiären Carbonsäuren werden dann mit Acetylen in Anwesenheit des Zinksalzes der Carbonsäuren (Encyl. Polym. Sci. Eng. 17, S.426-434) als Katalysator zu den entsprechenden Vinylestern umgesetzt oder diese werden durch Umesterung mit Essigsäurevinylester gewonnen (Ullmann, 4.Auflage, Band 19, S. 368 ff). Diese dienen als Copolymere, zum Beispiel für die Herstellung von modifizierten Polyvinylacetat, wo sie eine innere Weichmachung unter gleichzeitiger Erhöhung der Hydrolysestabilität bewirken. Auch hier wird durch den Einsatz der wenig verzweigten Sumpffraktion eine ganz wesentliche Verbesserung der Weichmachereigenschaften erzielt, wie anhand der Beispiele gezeigt wird.

Die weitgehend verzweigte Isomere enthaltende Kopffraktion hingegen wird mit besonderem Vorteil angewendet, wenn ein schneller biologischer Abbau nicht erwünscht ist oder wenn es auf besonders hohe Öllöslichkeit ankommt. Beides trifft in besonderem Maße auf die Metallsalze verzweigter Carbonsäuren zu, die als Trocknungsbeschleuniger in Farben und Lacken Einsatz finden. Hier bedeutet höhere Öllöslichkeit, daß höhere Konzentrationen des Metallsalzes im organischen Medium erzielbar sind. Da die Farben und Lacke den Untergrund schützen sollen, ist hier die Langlebigkeit ohne Zusatz von giftigen Mitteln essentiell wichtig.

Diese verzweigten Carbonsäuren können aus der Kopffraktion entweder durch Hydroformylierung und Oxidation der entstandenen Aldehyde oder vorzugsweise durch Hydrocarboxylierung erhalten werden. Hier bietet sich z. B. die KOCH-Synthese, wie in Falbe, New Synthesis with Carbon Monoxide, Springer Verlag, Berlin 1980, S. 372 beschrieben, an. Die höher verzweigten Olefine der Dimethylhexen-haltigen Fraktion liefern die unter den gewählten Reaktionsbedingungen stabilen Markownikow-Produkte, d. h. hauptsächlich tertiäre Carbonsäuren.

Die nach dem vorliegenden Verfahren hergestellten Dibutenfraktionen, insbesondere die verzweigte Olefine enthaltende Kopffraktion sind besonders für sauer katalysierte Reaktionen geeignet, bei denen Carbeniumionen als Zwischenstadium eine Rolle spielen, da sie sich besonders leicht aus verzweigten Olefinen bilden. Solche Reaktionen erfolgen dann in aller Regel mit verbesserter Selektivität gegenuber der Verwendung von wenig oder nicht verzweigten Olefinen.

Weiterhin kann die Dimethylhexen-haltige Fraktion nach Hydrierung als Vergaserkraftstoffkomponente mit hoher Octanzahl verwendet werden. Die folgenden Beispiele sollen die vorliegende Erfindung näher erläutern, ohne jedoch ihren Umfang zu beschränken.

**Beispiel 1 Dibuten-Fraktionierung**

Es wurde ein technisches Dibutengemisch mit einer Zusammensetzung gemäß Tabelle 4 fraktioniert.Die verwendete Kolonne wies folgende technische Daten auf:

| | |
|---|---|
| Gesamtlänge | 27,5 m |
| Durchmesser | DN 300 |
| Gewebepackungen | Sulzer CY (ca. 10 theoretische Böden je Meter) |
| Packungslänge | 18,72 m |

Insgesamt waren 117 Elemente der Gewebepackungen auf 9 Tragrosten eingebaut. Die Dampfverteilung innerhalb der Kolonne erfolgte durch 9 Rohrverteiler und 8 Flüssigkeitssammler. Die typischen Betriebsbedingungen waren:

| | |
|---|---|
| Zulauf | 100 l/h |
| Destillat | ~ 30 l/h |
| Sumpfablauf | ~ 70 l/h |
| Rücklauf | ~ 700 l/h |
| Kopftemperatur | 114 °C |
| Sumpftemperatur | 124 °C |
| Druckdifferenz | ~ 160 mbar |

Destillatabnahme, Zulauf und Rücklauf wurden mengengeregelt, der Sumpfablauf standgeregelt betrieben. Da der Zulauf direkt aus einer technischen Anlage erfolgte und infolgedessen die Konzentrationen geringfügig schwankten, wurde Kopf-und Sumpfabgang ständig gaschromatografisch analysiert und der Zulauf entsprechend geändert. Als Leitkomponenten hierfür dienten die Komponenten 15 und 16 des Gemisches. Es wurde so gefahren, daß Komponente 15 (trans-3,4-Dimethylhexen-2) möglichst vollständig im Destillat war, Komponente 16 (trans-5-Methylhepten-2) hingegen möglichst vollständig im Sumpf.
Die Kolonne wurde in einem 150 Tage dauernden Pilotbetrieb getestet. Dabei wurde festgestellt, daß es auch unter technischen Bedingungen möglich ist, die Kolonne reproduzierbar zu fahren. Die gesammelten Kopf- und Sumpffraktionen wurden nach Abschluß des Pilotbetriebs noch einmal gaschromatografisch untersucht, wobei die entstandenen Olefine direkt im Einspritzblock des Gaschromatografen hydriert wurden. Auf diese Weise erhält man eine Summenanalyse. Die Analyse des Eduktes, das sich geringfügig während dieser Zeit ändert, ist naturgemäß eine beispielhafte Einzelanalyse.

Folgende Ergebnisse wurden erhalten:

|  | Einsatz | Sumpfprodukt | Kopfprodukt |
|---|---|---|---|
| n-Octene | 16,8 % | 24,0 % | < 0,1 % |
| 3-Methyl-Heptene | 56,5 % | 65,3 % | 29,1 % |
| 3,4-Dimethylhexene | 24,7 % | 10,0 % | 63,5 % |
| 2,3-Dimethylhexene | 0,64 % | < 0,1 % | 1,6 % |
| 3,3-Dimethylhexene | 0,27 % | < 0,1 % | 1,0 % |
| 2,4-Dimethylhexene | 0,67 % | < 0,1 % | 2,5 % |
| Sonstige $C_8$-Olefine | 0,43 % | 0,7 % | 2,3 % |
| Isoindex | $\cong$1,18 | 0,87 | $\cong$ 1,71 |

**Tabelle 5:** Summenanalyse

**Beispiel 2 bis 4 Herstellung von tertiären Carbonsäuren**

Unfraktioniertes Dibuten (Beispiel 2), die erfindungsgemäß hergestellte Dimethylhexen-haltige Fraktion (Kopfprodukt, Beispiel 4) und die erfindungsgemäß hergestellte n-Octen-haltige Fraktion (Sumpfprodukt, Beispiel 3) wurden in Anlehnung an DE 23 39 947 zu tertiären Carbonsäuren umgesetzt. Als Katalysator diente ein Komplex aus Bor-Fluorid und Wasser und als Cokatalysator $Cu^+$. Die Umsetzungen erfolgten in einem Rührautoklaven in einem Temperaturbereich 20-35 °C und einem CO-Druck von 30 bar. Durch Nachdosieren von CO wurde der Druck konstant gehalten. Die Umsetzung wurde beendet, sobald keine CO-Aufnahme mehr zu beobachten war.

Nach Abtrennung von der Katalysatorphase, Wasserwäsche und destillativer Aufarbeitung der rohen Carbonsäuren wurden Produkte folgender Zusammensetzung erhalten (Werte in Massen-%).

Tabelle 6

| Produkt | Beispiel 2 | Beispiel 3 | Beispiel 4 |
|---|---|---|---|
|  | Carbonsäure A | Carbonsäure B | Carbonsäure C |
| 2,2-Dimethyl-Heptansäure | 7,31 | 2,30 | 8,17 |
| 2-Methyl-2-Ethyl-Hexansäure | 54,3 | 21,7 | 71,0 |
| 2-Methyl-2-Propyl-Pentansäure | 7.09 | 3,62 | 6,99 |
| 2,2-Diethyl-Pentansäure | 3,40 | 2,02 | 2,54 |
| 2,2,5-Trimethyl-Hexansäure | 0,83 | 1,71 | 0,09 |
| 2,2,4-Trimethyl-Hexansäure | 0,81 | 2,13 | 0,18 |
| 2,4-Dimethyl-2-Ethyl-Pentansäure | 1,76 | 4,44 | 0,30 |
| 2,2,3-Trimethyl-Hexansäure | 2,54 | 7,10 | 0,74 |
| 2-Methyl-2-Isopropyl-Pentansäure | 5,44 | 11,4 | 1,97 |
| 2,3-Dimethyl-2-Ethyl-PentansäureA | 7,27 | 18,9 | 3,40 |
| 2,3-Dimethyl-2-Ethyl-PentansäureB | 7,59 | 20,1 | 3,54 |

Tabelle 6 (fortgesetzt)

| Produkt | Beispiel 2 Carbonsäure A | Beispiel 3 Carbonsäure B | Beispiel 4 Carbonsäure C |
|---|---|---|---|
| 2-Ethyl-2-Isopropyl-Butansäure | 1,19 | 3,45 | 0,55 |
| Sonstige unbekannte Säuren | 0,50 | 1,13 | 0,48 |

**Beispiele 5 bis 7**

Die in Beispielen 2 bis 4 erhaltenen Gemische tertiärer Carbonsäuren wurden mit Acetylen bei Normaldruck bei einer Temperatur von 190-220 °C in Anwesenheit des Zinksalzes der jeweils umzusetzenden Säure nach der Gleichung umgesetzt:

$$R\text{-}COOH + HC \equiv CH \rightarrow R\text{-}COO\text{-}CH=CH_2$$

Die Reaktion wurde gemäß G.Hübner, Fette, Seifen, Anstrichmittel, 68, (4), S. 290-292 (1966) durchgeführt. Nach Destillation wurden Vinylester mit einer Reinheit von >= 99,8 % erhalten, die laut gaschromatografischer Untersuchung im wesentlichen die gleiche Isomerenzusammensetzung hatten wie die eingesetzten Carbonsäuren, im folgenden bezeichnet als Vinylester A (Basis Dibuten tel quel, Edukt: Carbonsäure A), Vinylester B (Basis Sumpfprodukt der Destillation Beispiel 1, Edukt: Carbonsäure B), Vinylester C (Basis Kopfprodukt der Destillation Beispiel 1, Carbonsäure C).

**Beispiele 8 bis 12**

Aus den Vinylestern gemäß Beispiel 5 bis 7 wurden Homopolymerisate nach folgender Standardvorschrift hergestellt (Beispiele 10 bis 12) und ihr Glaspunkt als Maß für die Eignung als Copolymere für innere Weichmachung ermittelt.

**<u>Einsatzstoffe</u>**

| Monomer | Gewichtsteile |
|---|---|
| Vinylester einer tertiären $C_9$- oder $C_{10}$-Carbonsäure | 100,00 |
| | |
| **Wäßrige Phase** | |
| VE-Wasser | 70,00 |
| Anionisches Tensid, z. B. Marlon® A 390 (85 % aktive Substanz) | 0,03 |
| Nichtionisches Tensid,z.B. Marlophen® 820 (25 %ige Lösung) | 8,00 |
| Kaliumperoxodisulfat ($K_2S_2O_8$) | 0,10 |
| Kaliumcarbonat | 0,25 |
| Hydroxyethylcellulose, z. B. Natrosol 250 L (oder LR) | 2,00 |
| Essigsäure (100%) | 0,20 |

**Durchführung**

Die wäßrige Phase und etwa 10 % des Monomeren wurden unter Rühren auf 75 °C erwärmt. Nach 15 Minuten bei dieser Temperatur wurden das restliche Monomere und die Initiatorlösung in getrennten Strömen zudosiert. Die Zugabe des Monomeren erfolgte gleichmäßig in 120 Minuten und die der Initiatorlösung in 135 Minuten. Während der Dosierung wurde die Temperatur zwischen 75 und 80 °C gehalten. Nach weiteren 120 minütigem Rühren bei der gleichen Temperatur wurde der Ansatz auf Raumtemperatur abgekühlt.

Aus der entstandenen Emulsion wurden, gegebenenfalls nach Filtration, Formkörper hergestellt, von denen durch Torsionschwingungsanalyse in Anlehnung an DIN 53455 die Glaspunkte bestimmt wurden.

Außerdem wurden zwei handelsübliche Vinylester, deren Glaspunkt bekannt ist, der gleichen Prozedur unterworfen, um die Vergleichbarkeit der Testprozedur sicherzustellen. Es handelte sich einmal um einen Vinylester aus tertiären $C_{10}$-Säuren (Basis Tripropen, Vergleichsbeispiel 8), der weit verbreitet als innerer Weichmacher für beispielsweise Vinylacetat (Glaspunkt laut Literatur - 3 °C) eingesetzt wird. Zum anderen wurde ein Vinylester aus tertiären $C_9$-Säuren eingesetzt (Vergleichsbeispiel 9), also ein Vinylester mit der gleichen Summenformel, jedoch unterschiedlichem Verzweigungsgrad wie die gemäß den vorstehenden Beispielen hergestellten Vinylester (Glaspunkt laut Literatur im Bereich + 60 °C).

Es wurden folgende Daten gemessen:

| Beispiel | 8 (Vergleich) | 9 (Vergleich) | 10 | 11 | 12 |
|---|---|---|---|---|---|
| Vinylester | C10-VE* | C9-VE* | A (Dibuten) | B (Sumpf) | C (Kopf) |

| Glaspunkt [°C] | - 3 | + 60 | - 3 | - 12 | + 15 |
|---|---|---|---|---|---|

**Tabelle 7:** * handelsübliche Vinylester auf der Basis von tertiären $C_9$- und $C_{10}$-Carbonsäuren (W.Lau, VeoVa, a Vinyl Ester Monomer, Polymers DotCom Magazine, 2, (2), Februar 1996)

Die Vergleichsbeispiele 8 und 9 zeigen, daß die Standardformulierung mit der Literatur vergleichbare Ergebnisse bringt. Außerdem zeigt Beispiel 9, daß normalerweise mit tertiären $C_9$-Carbonsäuren Vinylester mit außerordentlich hohem Glaspunkt erhalten werden, die zur inneren Weichmachung völlig ungeeignet sind.

Im Gegensatz hierzu zeigt schon der Vinylester auf Basis des eingesetzten Dibutens (Beispiel 10) eine weichmachende Eignung vergleichbar mit dem technisch üblichen Produkt (Vergleichsbeispiel 8). Der Vinylester auf Basis des Sumpfproduktes der Dibutendestillation (Beispiel 11) ist demgegenüber mit einem Glaspunkt von - 12 °C ein Comonomeres mit ganz wesentlich verbesserter weichmachender Wirkung. Selbst das Produkt aus den verzweigten tertiären Carbonsäuren (Beispiel 12; Basis Kopfprodukt der Destillation Beispiel 1) hat einen wesentlich geringeren Glaspunkt als das Vergleichsprodukt aus Beispiel 9. Wenn es auf die Anwendung als Comonomeres für innere Weichmachung ankommt, ist somit das Produkt aus Beispiel 11 im Vergleich zu den Produkten aus den Beispielen 8, 9, 10 und 12 mit Abstand am besten geeignet.

Die tertiären Carbonsäuren aus Beispiel 7 hingegen (Carbonsäure C, Basis Kopfprodukt Beispiel 1) ergeben nur gering weichmachende Comonomere (siehe Beispiel 12). Auf Grund ihrer stärkeren Verzweigung sind sie aber beispielsweise besonders gut geeignet für die Herstellung von öllöslichen Metallsalzen wie Trocknungsbeschleuniger für Lacke und Stabilisatoren für PVC oder für verseifungsstabile Ester als Lösemittel.

**Beispiele 13 bis 15**

Aus den Fraktionen gemäß Beispiel 1 wurden Phthalatweichmacher auf folgendem Weg hergestellt:

Die jeweilige Olefinfraktion (Einsatz, Sumpf- und Kopfprodukt) wurden wie aus der Literatur bekannt mit Synthesegas ($CO/H_2 \sim 1/1$) in Anwesenheit von Kobaltverbindungen als Katalysator bei ca. 200 bar und 180 °C hydroformyliert. Aus dem erhaltenen Reaktionsaustrag wurden die Wertprodukte ($C_9$-Aldehyde und $C_9$-Alkohole) destillativ abgetrennt und ebenfalls wie bekannt zu den Alkoholen hydriert. Das so erhaltene Isononanolgemisch wurde schließlich mit Phthalsäureanhydrid wie bekannt zu den entsprechenden Phthalsäureestern umgesetzt.

Die Prüfung für ihre Eignung als Weichmacher erfolgte über die Messung der Viskosität der Phthalsäureester. Die Viskosität der Ester nimmt bei gleicher Summenformel mit der Verzweigung ab; die weichmachenden Eigenschaften werden in umgekehrter Weise mit sinkender Verzweigung verbessert. In Tabelle 8 ist deutlich zu erkennen, daß aus dem Sumpfprodukt der Destillation gemäß Beispiel 1 ein besonders guter Weichmacher erhalten wird.

Im folgenden ist die Isomerenverteilung der bekannten $C_9$-Alkohole angegeben, mit denen ca. 98 % der gesamten Alkohole abgedeckt sind. Der Rest sind nicht aufgeklärte andere Isomere von $C_9$-Alkoholen. Die Viskosität bezieht sich auf den jeweils aus der Alkoholmischung hergestellten Phthalsäureester (gemessen bei 20 °C).

Tabelle 8

| Beispiel | 13 | 14 | 15 |
|---|---|---|---|
| **Olefin** | **Einsatz** | **Sumpf** | **Kopf** |
| n-Octene | 17,5 % | 25,0 % | 0,0 % |
| Methyl-Heptene | 54,7 % | 65,0 % | 31,0 % |
| Dimethyl-Hexene n-Nonanol | 27,7 % 8,4 % | 10,0 % 12,4 % | 69,0 % 0,0 % |
| **Isomerenverteilung der C$_9$-Alkohole** | 8,4 % | 12,4 % | 0,0 % |
| n-Nonanol | 4,6 % | 6,4 % | 0,0 % |
| 2-Methyl-Octanol | 2,5 % | 3,3 % | 0,0 % |
| 2-Ethyl-Heptanol | 2,0 % | 2,9 % | 0,0 % |
| 2-Propyl-Hexanol | 17,3 % | 20,5 % | 9,6 % |
| 4-Methyl-Octanol | | | |
| 2,3-Dimethyl-Heptanol | 1,8 % | 2,2 % | 1,2 % |
| 3-Ethyl-Heptanol | 7,0 % | 8,4% | 4,0 % |
| 2-Propyl-3-Ethyl-Pentanol | 0,2 % | 0,2 % | 0,1 % |
| 2-Ethyl-4-Methyl-Hexanol | 2,5 % | 2,8 % | 1,5 % |
| 2,5-Dimethyl-Heptanol | 8,2 % | 9,9 % | 4,7 % |
| 6-Methyl-Octanol | 17,5 % | 21,0 % | 9,9 % |
| 4,5-Dimethyl-Heptanol | 22,3 % | 8,1 % | 55,0 % |
| 3-Ethyl-4-Methyl-Hexanol | 5,4 % | 1,9 % | 14,0 % |
| Viskosität des Gemisches bei 20 °C in mPas | 77 | 68 | 103 |

Aus dem Sumpfprodukt der Dibutendestillation wird somit ein Weichmacher mit besonders niedriger Viskosität erhalten.

**Patentansprüche**

1.  Verfahren zur Fraktionierung von Dibutenen,
    dadurch gekennzeichnet,
    daß die Dibutene in eine n-Octen-haltige Fraktion mit einem Isoindex von weniger als 90 % und eine Dimethylhexen-haltige Fraktion mit einem Isoindex von mehr als 110 % des Isoindex der ursprünglichen Dibutene aufgetrennt werden.

2.  Verfahren nach Anspruch 1,
    dadurch gekennzeichnet,
    daß die n-Octen-haltige Fraktion einen Isoindex von weniger als 1,0 aufweist.

3.  Verfahren nach Anspruch 1,
    dadurch gekennzeichnet,
    daß die n-Octen-haltige Fraktion einen Isoindex von weniger als 0,9 aufweist.

4.  Verfahren nach einem der Ansprüche 1 bis 3,
    dadurch gekennzeichnet;

daß die Fraktionierung der Dibutene durch kontinuierliche Destillation erfolgt.

5. Verfahren nach Anspruch 4,
dadurch gekennzeichnet,
daß die Destillation bei Normaldruck ausgeführt wird.

6. Verfahren nach einem der Ansprüche 1 bis 5,
dadurch gekennzeichnet,
daß die n-Octen-haltige Fraktion als Sumpfprodukt und die Dimethylhexenhaltige Fraktion als Kopfprodukt erhalten werden.

7. Verfahren nach einem der Ansprüche 1 bis 6,
dadurch gekennzeichnet,
daß die n-Octen-haltige Fraktion bei Normaldruck einen Siedebereich von 110 bis 126 °C aufweist.

8. Verfahren nach Anspruch 7,
dadurch gekennzeichnet,
daß die n-Octen-haltige Fraktion bei Normaldruck einen Siedebereich von 115 bis 123 °C aufweist.

9. Verfahren nach einem der Ansprüche 1 bis 6,
dadurch gekennzeichnet,
daß die Dimethylhexen-haltige Fraktion bei Normaldruck einen Siedebereich von 95 bis 120 °C aufweist.

10. Verfahren nach Anspruch 9,
dadurch gekennzeichnet, daß die Dimethylhexen-haltige Fraktion bei Normaldruck einen Siedebereich von 105 bis 115 °C aufweist.

11. Verwendung der Dimethylhexen-haltigen Fraktion gemäß den Ansprüchen 1 bis 6, 9 und 10 zur Herstellung von Nonansäuren.

12. Verwendung der Dimethylhexen-haltigen Fraktion gemäß den Ansprüchen 1 bis 6, 9 und 10 zur Herstellung von Isononanol.

13. Verwendung der n-Octen-haltigen Fraktion gemäß den Ansprüchen 1 bis 8 zur Herstellung von Isononanol.

14. Verwendung der n-Octen-haltigen Fraktion gemäß den Ansprüchen 1 bis 8 zur Herstellung von Nonansäuren.

15. Verwendung der Dimethylhexen-haltigen Fraktion gemäß den Ansprüchen 1 bis 6, 9 und 10 nach Hydrierung als Kraftstoffkomponente.

**Europäisches**
**Patentamt**

**ERKLÄRUNG**

die nach Regel 45 des Europäischen Patent-
Übereinkommens für das weitere Verfahren als
europäischer Recherchenbericht gilt

Nummer der Anmeldung

EP 99 12 6213

| Die Recherchenabteilung ist der Auffassung, daß die vorliegende Patentanmeldung den Vorschriften des EPÜ in einem solchen Umfang nicht entspricht, daß sinnvolle Ermittlungen über den Stand der Technik auf der Grundlage aller Patentansprüche nicht möglich sind. | KLASSIFIKATION DER ANMELDUNG (Int.Cl.7) |
|---|---|
| Grund: | C07C11/02 |

Die geltenden Patentansprüche 1 bis 15 beziehen sich auf ein Produkt charakterisiert durch eine erstrebenswerte Eigenheit oder Eigenschaft, nämlich der Isoindex der Fraktionen. Die Patentansprüche umfassen daher alle Produkte die diese Eigenheit oder Eigenschaft aufweisen, wohingegen die Patentanmeldung durch die Beschreibung im Sinne von Art. 83 EPÜ nur für eine begrenzte Zahl solcher Produkte gestützt wird bzw. fehlt der Patentanmeldung die nötige Offenbarung in einem solchen Masse, dass eine sinnvolle Recherche über den gesamten erstrebten Schutzbereich unmöglich erscheint. Desungeacht fehlt den Patentansprüchen auch die in Art. 84 EPÜ geforderte Klarheit, nachdem in ihnen versucht wird, das Produkt über das jeweils erstrebte Ergebnis zu definieren. Auch dieser Mangel an Klarheit ist dergestalt, dass er eine sinnvolle Recherche über den gesamten erstrebten Schutzbereich unmöglich macht. Ausserdem sind die geltenden Patentansprüche 1 bis 15 auf ein Produkt, das mittels folgendem Parameter definiert wird, zu beziehen: der Isoindex der Fraktionen.

Die Verwendung dieses Parameters muss in gegebenen Zusammenhang als Mangel an Klarheit im Sinne van Art. 84 EPÜ erscheinen. Es ist unmöglich, die von Anmelder gewählten Parameter mit dem zu vergleichen, was der Stand der Technik hierzu offenbart. Der Mangel an Klarheit ist dergestalt, dass er eine sinnvolle

-/--

| Recherchenort | Abschlußdatum | Prüfer |
|---|---|---|
| DEN HAAG | 3. Mai 2000 | Van Geyt, J |

**Europäisches Patentamt**

**ERKLÄRUNG**

die nach Regel 45 des Europäischen Patent-Übereinkommens für das weitere Verfahren als europäischer Recherchenbericht gilt

Nummer der Anmeldung

EP 99 12 6213

| Die Recherchenabteilung ist der Auffassung, daß die vorliegende Patentanmeldung den Vorschriften des EPÜ in einem solchen Umfang nicht entspricht, daß sinnvolle Ermittlungen über den Stand der Technik auf der Grundlage aller Patentansprüche nicht möglich sind.<br><br>Grund:<br><br>vollständige Recherche unmöglich macht.<br><br>Der Anmelder wird darauf hingewiesen, daß im Zuge der Prüfung eine Recherche durchgeführt werden kann, sollten die einer Erklärung gemäß Regel 45 EPÜ zugrundeliegenden Mängel behoben worden sein (Vgl. EPA-Richtlinien C-VI, 8.5).<br><br>---<br>----- | **KLASSIFIKATION DER ANMELDUNG (Int.Cl.7)** |
|---|---|

| Recherchenort | Abschlußdatum | Prüfer |
|---|---|---|
| DEN HAAG | 3. Mai 2000 | Van Geyt, J |

EPO FORM 1504 (P04C39)